# EUROPEAN PATENT SPECIFICATION

(11) **EP 1 755 650 B1**
(45) Date of publication and mention of the grant of the patent: **09.07.2008**
(21) Application number: 05753131.1
(22) Date of filing: 23.05.2005
(51) Int. Cl.: A61K 38/21, A61K 9/16, A61K 9/00, A61K 47/18

(54) **A BIOMOLECULE-CONTAINING SUSPENSION FORMULATION OF INCREASED STABILITY DELIVERABLE VIA AN IMPLANTABLE DELIVERY DEVICE**
EINE BIOMOLEKÜLE ENTHALTENDE SUSPENSIONSFORMULIERUNG VON ERHÖHTER STABILITÄT - FREISETZBAR ÜBER EIN IMPLANTIERBARES HILFSMITTEL
UNE SUSPENSION CONTENANT UNE BIOMOLÉCULE - D'UNE STABILITÉ AUGMENTÉE ET D'UNE ADMINISTRATION PAR UN DISPOSITIF IMPLANTABLE

(30) Priority: 25.05.2004 US 574662 P; 03.02.2005 US 650226 P
(43) Date of publication of application: 28.02.2007
(73) Proprietor: Intarcia Therapeutics, Inc., Emeryville CA 94545 (US)
(72) Inventor: JUNNARKAR, Gunjan, Palo Alto, California 94303 (US); DESJARDIN, Michael A., Sunnyvale, CA 94087 (US); LIU, Kui, Redwood City, CA 94065 (US); LI, Zengji, San Ramon, CA 94583 (US)
(74) Representative: Walton, Seán Malcolm
(86) International application number: PCT/US2005/017914
(87) International publication number: WO 2005/115333

(56) References cited:
- WO-A-20/04056338
- US-A- 5 219 572
- US-A1- 2003 044 467
- US-A1- 2003 059 376
- US-A1- 2003 215 515
- US-B1- 6 468 961

## Description

### BACKGROUND OF THE INVENTION

The invention relates generally to formulations deliverable via sustained release systems, such as implantable drug delivery devices and depot injections.

Interferons are a group of glycoprotein cytokines produced by cells in response to various stimuli, such as exposure to virus, bacterium, parasite, or other antigen. Interferons have antiviral, immunomodulatory, and antiproliferative activities. Interferons are classified as Type I or Type II. Interferons classified as Type I bind to a common receptor called the Interferon Type I or α-β receptor and are produced by leukocytes, fibroblasts, or lymphoblasts in response to virus or interferon inducers. Interferon Type I includes interferon alpha (IFN-α), interferon beta (IFN-β), and interferon omega (IFN-ω), but IFN-ω has limited homology to human IFN-α (about 60%) and human IFN-β (about 29%). Interferons classified as Type II are produced by T-lymphocytes. Interferon Type II includes interferon gamma (IFN-γ). Interferons are used for treatment of viral hepatitis, multiple sclerosis, and certain cancers. IFN-ω in particular has been indicated for treatment of Hepatitis B & C populations. The injectable form of IFN-ω is currently in Phase II clinical studies. This injectable form is solution-based and is not formulated for sustained delivery.

There is interest in delivering interferons to patients in a controlled manner over a prolonged period without intervention. For instance, sustained delivery of IFN-ω can improve the therapeutic effect of IFN-ω by reduction or elimination of peak plasma-level related effects of multiple bolus injections, thereby potentially minimizing systemic side effects such as fatigue and flu-like symptoms. Sustained delivery of a beneficial agent without intervention can be provided by implantable drug delivery devices, e.g., osmotic, mechanical, or electromechanical pump implants, and depot injections. Implantable drug delivery devices are attractive for a number of reasons. For example, implantable drug delivery devices can be designed to provide therapeutic doses of the drug over periods of weeks, months, or even a year. Depot injections typically provide therapeutic doses over periods of weeks. Implantable drug delivery devices once inserted therapeutic doses over periods of weeks. Implantable drug delivery devices once inserted in the patient are not easily tampered with by the patient. Thus, patient compliance is generally assured.

Sustained delivery of an interferon requires the interferon to be contained within a formulation that is substantially stable at elevated temperature, e.g., 37°C or higher, over the operational life of the implantable delivery drug device. Interferon is a biomolecule, specifically a protein. Generally speaking, protein formulations that are stable at elevated temperature for a long duration, e.g., weeks, months, or a year, are difficult to design. Proteins are naturally active in aqueous environments. Therefore, it would be convenient to formulate proteins as aqueous solutions. Unfortunately, proteins are typically only marginally stable in aqueous formulations for a long duration. One reason for this is that proteins can degrade via a number of mechanisms, such as deamidation (usually by hydrolysis), oxidation, disulfide interchange, and racemization, and water is a reactant in many of these degradation pathways. Water also acts as a plasticizer and facilitates denaturation and/or aggregation of protein molecules.

Aqueous protein formulations can be reduced to dry particle protein formulations using drying techniques such as freeze-drying (or lyophilization), spray-drying, and dessication. Such dry particle protein formulations can exhibit significantly increased stability over time at ambient and even elevated temperature. However, it is difficult to controllably deliver dry particle formulations from an implantable drug delivery device at a desired flow rate. It has been suggested to suspend the dry particle protein formulation in a non-aqueous, flowable vehicle. Preferably, the suspension vehicle has a high viscosity, e.g., 1 kP or more, so that the particles are substantially uniformly dispersed in the suspension for a desired duration. Further, the suspension formulation should be stable at storage and delivery conditions for the desired duration and maintain its flowability for the operational life of the implantable drug delivery device.

Non-aqueous suspension vehicles for delivering beneficial agents via implantable drug delivery devices have been described in literature. For example, U.S. Patent No. 5,904,935 (Eckenhoff et al.) teaches non-aqueous suspension vehicles that include waxes having a softening temperature at or less than body temperature, hydrogenated vegetable oils, silicon oil, medium chain fatty acid monoglycerides, and polyols. The viscosity of these suspension vehicles can be increased to a desired level using thickening agents such as hydrogels, such as cellulose ethers, e.g., hydroxypropyl cellulose and povidone. U.S. Patent No. 6,264,990 (Knepp et al.) discloses non-aqueous, anhydrous, aprotic, hydrophobic, non-polar suspension vehicles with low reactivity. Examples of such vehicles include perfluorodecalin, methoxyflurane, and perfluorotributylamine. Polymeric materials, such as polyvinylpyrrolidone (PVP), may also be used as suspension vehicles.

U.S. Patent Publication No. US-2004-0224903-A1, discloses suspension vehicles made of single-phase, viscous, flowable compositions that are substantially formed of hydrophobic, non-polymeric materials. Non-polymeric materials used in forming these suspension vehicles include, but are not limited to, hydrophobic saccharide materials, organogels, or lipid materials that behave as single-phase vehicles. According to the publication, exemplary saccharide materials that may be used in formulating a suspension vehicle include, but are not limited to, substituted sucrose esters that exist as fluids at ambient or physiological temperatures, such as sucrose acetate isobutyrate (SAIB). These non-polymeric materials allow the formulation of protein suspensions that are not only stable at ambient and physiological temperatures but are also capable of maintaining substantially uniform dispersion of protein particles.
US5219572 discloses a controlled release delivery device for implantation comprising a plurality of beadlets which are based on a core matrix (macromolecular protein, such as interferon, stabilizers, binders, surfactants, preservatives, arginine, sucrose as vehicle).
In US2003059376 a systemic delivery of a protein through the buccal mucosa is described, wherein the suspension comprises dehydrated solid particles in a delivery medium and these particles comprise a surfactant, a protein, at least one buffer, at least one surfactant, a membrane-permeation enhancer. The delivery medium (seen as vehicle) includes a propellant and ethanol.
The powder particles of US2003215515 contain a bioactive material. The suspension comprises the bioactive material and a polyol (sucrose and other sugar derivatives, a polymer), a surfactant (polyethylene glycol sorbitan monolaurate etc), and an amino acid.
US2003044467 refers to an implantation system which comprises a beneficial agent dissolved or dispersed substantially throughout a viscous gel (based on a biocompatible polymer and a solvent, an emulsifying agent, a pore former, a solubility modulator for the beneficial agent and an osmotic agent).
An implantable composition for the sustained delivery of a beneficial agent (interferon) comprising a polylactide polymer, an amount of a solvent (lower alkyl or aralkylester of benzoic acid), an emulsifying agent, an osmotic agent is shown in US6468961.
W02004056338 describes an implantable pump comprising a pharmaceutical formulation which itself comprises a single phase vehicle that contains a hydrophobic, non-polymeric material (hydrophobic non-polymeric material selected from hydrophobic saccharide materials, organogels, lipid materials, SAIB) and beneficial agent particles (interferon amongst others). The viscous, gel-like suspension vehicle may include an amount of other excipients or adjuvants, such as surfactants, antioxidants, stabilizers and viscosity modifiers.

Hydrophobic vehicles, such as SAIB, particularly when used without added excipients, can behave like a depot in the presence of a hydrophilic medium. This means that the protein suspended in the vehicle would not be instantaneously released from the vehicle in the presence of the hydrophilic medium. For depot injection applications, the depot effect of the suspension vehicle is typically desirable. For implanted delivery devices, when the suspension vehicle behaves like a depot in the release medium, control of release by the suspension vehicle is cumulative to the control of release by the delivery device. This additional control of release by the suspension vehicle may or may not be desirable depending upon the application. Anyhow, non-instantaneous release of the protein from the suspension vehicle would only be acceptable if the protein is stable in the vehicle in the presence of the release medium and during transition from the suspension vehicle into the release medium.

From the foregoing, there continues to be a desire for improved stable formulations of biomolecules, particularly proteins, more particularly interferons, that are deliverable via a sustained delivery system, such as an implantable drug delivery device or depot injection.

### SUMMARY OF THE INVENTION

In one aspect, the invention relates to a suspension formulation for therapeutic use which comprises a non-aqueous, hydrophobic vehicle exhibiting viscous fluid characteristics, a dry particle formulation comprising a biomolecule dispersed in the vehicle, and a surfactant incorporated in at least one of the hydrophobic vehicle and dry particle formulation.

In another aspect, the invention relates to a dry particle formulation comprising an interferon, a buffer, a surfactant, and one or more stabilizers selected from the group consisting of a carbohydrate, an antioxidant, and an amino acid.

In yet another aspect, the invention relates to an implantable delivery device which comprises a suspension formulation comprising a non-aqueous, hydrophobic vehicle exhibiting viscous fluid characteristics, a dry particle formulation comprising an interferon dispersed in the vehicle, and a surfactant incorporated in at least one of the vehicle and dry particle formulation. The implantable delivery device further includes a reservoir containing the suspension formulation in an amount sufficient to provide continuous delivery of the interferon in a therapeutically effective dose in an environment of use over at least one month.

In another aspect, the invention relates to a method of enhancing release of interferon omega in a hydrophilic release rate medium which comprises suspending a dry particle formulation of interferon omega in a non-polymeric, hydrophobic vehicle and incorporating a surfactant in at least one of the dry particle formulation and the hydrophobic vehicle.

Other features and advantages of the invention will be apparent from the following description.

### BRIEF DESCRIPTION OF DRAWINGS

FIG. 1 shows a scanning electron microscope (SEM) image of spray dried IFN-ω particles according to one embodiment of the invention.

FIG. 2 shows SEM image of IFN-ω particles spray dried with Pluronic F68.

FIG. 3 shows fraction of IFN-ω recovered from aqueous phase over time at 37°C.

FIG. 4 shows total BFN-ω recovered from aqueous and solid phases over time at 37°C.

### DETAILED DESCRIPTION OF THE INVENTION

The invention will now be described in detail with reference to a few preferred embodiments, as illustrated in accompanying drawings. In the following description, numerous specific details are set forth in order to provide a thorough understanding of the invention. However, it will be apparent to one skilled in the art that the invention may be practiced without some or all of these specific details. In other instances, well-known features and/or process steps have not been described in detail in order to not unnecessarily obscure the invention. The features and advantages of the invention may be better understood with reference to the drawings and discussions that follow.

The invention provides formulations including biomolecules that are deliverable via sustained delivery systems, in particular implantable drug delivery devices and possibly depot injections. Biomolecules considered herein are those that may provide a therapeutic benefit to an animal or human subject and exhibit increased stability when formulated in a non-aqueous suspension. Biomolecules considered herein are generally degradable in water but generally stable as dry particles at ambient and physiological temperatures. Examples of biomolecules include, but are not limited to, peptides, polypeptides, proteins, amino acids, nucleotides, polymers of amino acid residues or nucleotide residues, hormones, viruses, antibodies that are naturally derived, synthetically produced, or recombinantly produced, conjugated proteins, such as lipoproteins and post translationally modified forms, e.g., glycosylated proteins, and proteins having D-amino acids, modified, derivatized or non-naturally occurring amino acids in the D- or L-configuration and/or peptomimetic units as part of their structure.

Specific examples of biomolecules that may provide a therapeutic effect include, but are not limited to, baclofen, GDNF, neurotrophic factors, conatonkin G, Ziconotide, clonidine, axokine, anitsense oligonucleotides, adrenocorticotropic hormone, angiotensin I and II, atrial natriuretic peptide, bombesin, bradykinin, calcitonin, cerebellin, dynorphin N, alpha and beta endorphin, endothelin, enkephalin, epidermal growth factor, fertirelin, follicular gonadotropin releasing peptide, galanin, glucagon, gonadorelin, gonadotropin, goserelin, growth hormone releasing peptide, histrelin, insulin, interferons, leuprolide, LHRH, motilin, nafarerlin, neurotensin, oxytocin, relaxin, somatostatin, substance P, tumor necrosis factor, triptorelin, vasopressin, growth hormone, nerve growth factor, blood clotting factors, ribozymes, and antisense oligonucleotides. Analogs, derivatives, antagonists, agonists, and pharmaceutically acceptable salts of each of the above mentioned agents may also be used in formulations of the invention.

Of particular interest in this invention are interferons. The interferons may be recombinant molecules that can activate the Interferon Type I receptor (α-β receptor) or Interferon Type II receptor. These recombinant molecules may or may not contain sequence homology to native human Type I or Type II interferons. Interferons according to embodiments of the invention may be selected from the group consisting of proteins having the biological activity of recombinant human interferon, interferon analogs, interferon isoforms, interferon mimetics, interferon fragments, hybrid interferon proteins, fusion protein oligomers and multimers of the above, homologues of the above, glycosylation pattern variants of the above, muteins of the above, and interferon molecules containing the minor modifications enumerated above. Interferons according to the invention shall not be limited by method of synthesis or manufacture and shall include those synthesized or manufactured by recombinant (whether produced from cDNA or genomic DNA), synthetic, transgenic, and gene-activated methods. Specific examples of interferons include, but are not limited to, IFN-α, IFN-β, IFN-ω, and IFN-γ.

Embodiments of the invention provide dry particle formulations including biomolecules. Dry particle formulations of the invention have a low moisture content, typically less than 5 wt%. In accordance with one embodiment of the invention, a dry particle formulation includes an interferon as described above. The dry particle interferon formulation also includes stabilizers. In one embodiment, the stabilizers include a carbohydrate, an antioxidant and/or amino acid. The dry particle interferon formulation also includes a buffer. The amounts of stabilizers and buffer in the dry particle formulation can be determined experimentally based on the activities of the stabilizers and buffers and the desired characteristics of the formulation. In one embodiment, the amount of carbohydrate in the formulation is determined by aggregation concerns. In general, the carbohydrate level should not be too high so as to avoid promoting crystal growth in the presence of water due to excess carbohydrate unbound to interferon. In one embodiment, the amount of antioxidant in the formulation is determined by oxidation concerns. In one embodiment, the amount of amino acid in the formulation is determined by oxidation concerns and/or formability of particles during spray drying. In one embodiment, the amount of buffer in the formulation is determined by pre-processing concerns, aggregation concerns, and formability of particles during spray drying. The buffer may stabilize interferon during processing, e.g., spray drying, when all excipients are solubilized. In general, too much buffer can produce a buffer system in the presence of water, which can then lead to crystallization.

Examples of carbohydrates that may be included in the dry particle formulation include, but are not limited to, monosaccharides, such as fructose, maltose, galactose, glucose, D-mannose, and sorbose, disaccharides, such as lactose, sucrose, trehalose, cellobiose, polysaccharides, such as raffinose, melezitose, maltodextrins, dextrans, and starches, and alditols (acyclic polyols), such as mannitol, xylitol, maltitol, lactitol, xylitol sorbitol, pyranosyl sorbitol, and myoinsitol. Preferred carbohydrates include non-reducing sugars, e.g., sucrose, trehalose, mannitol, and dextrans.

Examples of antioxidants that may be included in the dry particle formulation include, but are not limited to, methionine, ascorbic acid, sodium thiosulfate, catalase, platinum, ethylenediaminetetraacetic acid (EDTA), citric acid, cysteins, thioglycerol, thioglycolic acid, thiosorbitol, butylated hydroxanisol, butylated hydroxyltoluene, propyl gallate.

Examples of amino acids that may be included in the dry particle formulation include, but are not limited to, arginine, methionine, glycine, histidine, alanine, L-leucine, glutamic acid, Iso-leucine, L-threonine, 2-phenylamine, valine, norvaline, praline, phenylalanine, trytophan, serine, asparagines, cysteine, tyrosine, lysine, and norleucine. Preferred amino acids include those that readily oxidize, e.g., cysteine, methionine, and trytophan.

Examples of buffers that may be included in the dry particle formulation include, but are not limited to, citrate, histidine, succinate, phosphate, maleate, tris, acetate, carbohydrate, and gly-gly. Preferred buffers include citrate, histidine, succinate, and tris.

The dry particle formulation may include other excipients selected from, for example, surfactants, bulking agents, and salts. Examples of surfactants include, but are not limited to, Polysorbate 20, Polysorbate 80, Tween 20, Tween 80, Pluronic F68, and sodium docecyl sulfate (SDS). Examples of bulking agents include, but are not limited to, mannitol and glycine. Examples of salts include, but are not limited to, sodium chloride, calcium chloride, and magnesium chloride. Possible advantages of incorporating a surfactant into the dry particle formulation will be further discussed in this disclosure.

Table 1 below shows protein particle formulation composition ranges according to some embodiments of the invention. In one embodiment, a dry particle interferon formulation includes 1:2:1:1.5-2.5 interferon: carbohydrate: antioxidant and/or amino acid: buffer. One example of a dry particle interferon formulation is 1:2:1:1.5-2.5 IFN-ω: sucrose: methionine: citrate. In another specific example, a dry particle interferon formulation includes 1:2:1:1.5-2.5:0.06 interferon: carbohydrate: antioxidant and/or amino acid: buffer: surfactant.

**TABLE 1**

| Loading in dry particle formulation (wt%) | Range | Preferred Range | Most Preferred Range |
|---|---|---|---|
| Protein | 0.1 to 99.9% | 1 to 50% | 1 to 30% |
| Surfactant | 0.0 to 10% | 0.01 to 10% | 0.01 to 5% |
| Bulking Agent | 0 to 99.9% | 0 to 70% | |
| Salt | 0 to 99.9% | 0 to 70% | |
| Stabilizers to protein (wt ratio) | Range | Preferred Range | Most Preferred Range |
| Carbohydrate | 0.1 to 99.9 | > 0.5 | > 1 |
| Antioxidant and/or amino acid | 0.1 to 99.9 | > 0.5 | |
| Buffer | Range | Preferred Range | Most Preferred Range |
| Buffer Concentration | 5 mM to 50 mM | 5 mM to 25 mM | |
| Buffer pH | 5.0 to 8.0 | | |

Dry particle formulations according to embodiments of the invention may be prepared by spray drying, lyophilization, or other technique available in the art for forming particles from a mixture of components. A typical spray dry process may include loading a spray solution containing a protein and stabilizing excipients into a sample chamber, which may be maintained at refrigeration to room temperature. Refrigeration generally promotes stability of the protein. A feed pump then sprays the spray solution into a nozzle atomizer. At the same time, atomized gas (typically, air, nitrogen, or inert gas) is directed at the outlet of the nozzle atomizer to form a mist of droplets from the spray solution. The mist of droplets are immediately brought into contact with a drying gas in a drying chamber. The drying gas removes solvent from the droplets and carries the dry particles into a collection chamber.

Suspension formulations according to embodiments of the invention are prepared by incorporating dry particle formulations according to embodiments of the invention into non-aqueous, hydrophobic vehicles. The non-aqueous, hydrophobic vehicles may be any combination of solvent, liquid or non-liquid polymer, liquid or non-liquid non-polymer, and surfactant.

In one embodiment, a non-aqueous, hydrophobic vehicle used in a suspension formulation of the invention is biodegradable, i.e., it disintegrates or breaks down over a period of time in response to a biological environment. This breakdown may take place by one or more physical or chemical processes, such as by enzymatic action, oxidation, reduction, hydrolysis (e.g., proteolysis), displacement, or dissolution by solubilization, emulsion or micelle formation. In one embodiment, the components of the vehicle are selected such that the vehicle has a viscosity in a range from 1 kP to 1,000 kP, preferably 5 kP to 250 kP, more preferably 5 kP to 30 kP. In one embodiment, to maintain stability of the biomolecule at elevated temperature, e.g., 37°C or higher, over a time period, the components of the vehicle are chosen such that the vehicle does not react with the biomolecule. The components of the vehicle may be chosen such that the vehicle has little or no solubility for the selected biomolecule and particle excipients, thereby maintaining the selected biomolecule and excipients as dry particles, thereby achieving stability of the selected biomolecule.

In another embodiment, a suspension formulation is made by suspending a dry particle formulation according to an embodiment of the invention in a non-aqueous, single-phase, hydrophobic vehicle including a non-polymer. Examples of non-polymeric materials suitable for use include, but are not limited to, hydrophobic saccharide materials, organogels, or lipid materials that behave as single-phase vehicles, e.g., lipid gels such as dioleoyl phisphatidylcholine (DOPC). Exemplary saccharide materials include, but are not limited to, sucrose esters that exist as fluids at ambient or physiological temperature, such as sucrose acetate isobutyrate (SAIB). The vehicle may or may not include one or more solvents. For example, SAIB, a liquid non-polymer, can be used "neat," i.e., without addition of other excipients. Examples of solvents for creating lipid gel vehicle (with DOPC) include, but are not limited to, n-methyl propanol, cottonseed oil, sesame oil, soybean oil, vitamin E, castor oil, Polysorbate 80, and N dimethylacetamide.

The non-aqueous, single-phase, hydrophobic vehicle described above may also include excipients such as surfactants, preservatives, and stabilizers. Surfactants may be included in the vehicle to facilitate release of the biomolecule from the vehicle once the formulation is delivered to an environment of use or to help maintain stability of the biomolecule when the biomolecule is suspended in the vehicle. Where included, surfactants will typically account for less than 20 wt%, preferably less than 10 wt%, more preferably less than 5 wt% of the vehicle. Generally, preservatives are included in the vehicle only in amounts sufficient to achieve the desired preservative effect. Examples of surfactants that may be used in the vehicle include, but are not limited to, Tweens, Pluronics, Span 20, Span 40, Span 60, Span 80, glyceryl caprylate, glyceryl laurate, PEG-8 caprylic capric glycerides, polyglyceryl-6 oleate, dioctyly sodium, sulfosuccinate, and Vitamin E TPGS. Preservatives that may be used in the vehicle include, for example, antioxidants and antimicrobial agents. Examples of potentially useful antioxidants include, but are not limited to, tocopherol (vitamin E), ascorbic acid, ascorbyl palmitate, butylated hydroxyanisole, butylated hydroxytoulene, and propyl gallate.

In one embodiment, a suspension formulation according to an embodiment of the invention includes a dry particle interferon formulation, as described above, suspended in a non-aqueous, hydrophobic vehicle. Varying amounts of the dry particle interferon formulation may be loaded into the vehicle to provide a formulation that allows dosing of the interferon at a desired rate over a chosen time period. The suspension formulation may include 0.1 to 40 wt%, preferably 0.1 to 20 wt%, of an interferon. The suspension formulation may include greater than 60 wt%, preferably greater than 80 wt%, of the suspension vehicle.

Suspension formulations according to embodiments of the invention may be formulated for delivery from an implantable drug delivery device or for use as a depot injection. The implantable drug delivery device may be embodied by any such device capable of delivering a flowable formulation at a controlled rate over a sustained period after implantation within a subject. One example of a suitable implantable drug delivery device is an osmotic pump implant, such as available under the trade name DUROS® implant. Non-osmotic pump implants may also be used. The suspension formulation may be formulated for delivery at flow rates up to 5 ml/day, depending on the biomolecule to be delivered and the implantable drug delivery device used to deliver the suspension formulation. Where the biomolecule is delivered from an osmotic pump implant designed to provide low flow rates, the formulation is preferably formulated for delivery of between 0.5 and 5 pL/day, with flow rates of about 1.5 µL/day and 1.0 µL/day being particularly preferred. In one embodiment, a suspension formulation according to an embodiment of the invention is formulated to deliver interferon from an implanted device in a range from 1 ng/day to 600 µg/day over one month, preferably over three months, more preferably over one year.

As previously discussed, non-aqueous, hydrophobic vehicles can behave like a depot when released into a hydrophilic medium. This depot effect may or may not be desirable depending on the application. However, where the depot effect is desirable or acceptable, it is important that the biomolecule suspended in the hydrophobic vehicle remains stable in the hydrophobic vehicle in the presence of the hydrophilic medium and during release from the hydrophobic vehicle into the hydrophilic medium.

The inventors have found that addition of a small amount of surfactant directly into dry particle formulations of the invention and/or into hydrophobic vehicles incorporating the dry particle formulations of the invention can enhance stability of the biomolecules in the dry particle formulations as the dry particle formulations are released from the hydrophobic vehicles into the hydrophilic media. While not wishing to be bound by theory, the inventors believe that addition of the small amount of surfactant directly into the dry particle formulations or suspension vehicles of the invention may have modified the interfacial behavior of the biomolecules in the dry particle formulations, leading to reduction in denaturation and aggregation of the biomolecules as the biomolecules transition from a hydrophobic vehicle into a hydrophilic medium. In particular, the surfactants may have helped to form particles having more hydrophobic excipients on the outside and more hydrophilic excipients on the inside. This biomolecular distribution may have played an important role in biomolecule stability during release from the hydrophobic vehicle into the hydrophilic medium.

Surfactants that may be incorporated in dry particle formulations and/or suspension vehicles according to embodiments of the invention may be ionic or nonionic. Some examples of surfactants include, but are not limited to, Polysorbate 20, Polysorbate 80, Tweens, Pluronic F68, sodium docecyl sulfate (SDS), Span 20, Span 40, Span 60, Span 80, Vitamin E TPGS, glyceryl caprylate, glyceryl laurate, PEG-8 caprylic capric glycerides, polyglyceryl-6 oleate, Pluronics, and dioctyly sodium sulfosuccinate. Table 2 below shows surfactant loading for dry particle formulations and suspension vehicles according to some embodiments of the invention.

**TABLE 2**

| | Suspension formulation | | Surfactant Loading in | |
|---|---|---|---|---|
| | Suspension Vehicle | Dry particle formulation | Dry particle formulation | Vehicle |
| Range | > 60 wt% | 0.1 to 40 wt% | 0.01 to 10 wt% | 0.01 to 20 wt% |
| Preferred Range | > 80 wt% | 0.1 to 20 wt% | 0.01 to 5 wt% | |

A study was conducted to assess the effect of surfactant on the release of dry particle interferon formulations according to embodiments of the invention from hydrophobic vehicles into hydrophilic release rate media. In the study, the hydrophobic vehicle is SAIB and the hydrophilic release rate medium is phosphate buffer solution. SAIB is a high viscosity, hydrophobic liquid with limited water solubility. It has a viscosity of approximately 3.2 kP at 37°C. SAIB is produced by the controlled esterification of natural sugar (sucrose) with acetic and isobutyric anhydrides. The materials used for the study are listed in Table 3 below.

**TABLE 3**

| MATERIAL | SOURCE |
|---|---|
| Pluronic F68 | BASF |
| Span 40 | Aldrich |
| Spray dried IFN-ω: Sucrose: Methionine: Citrate (1:2:1:2.15, 25 mM citrate buffer) | |
| Spray dried IFN-ω: sucrose: Methionine: Citrate: 1% Pluronic F68 (1:2:1:2.15:0.06, 25 mM citrate buffer) | |
| SAIB | Eastman Chemical Company |
| Phosphate Buffer Solution (PBS) with 0.2% Na Azide | |

### EXAMPLE 1

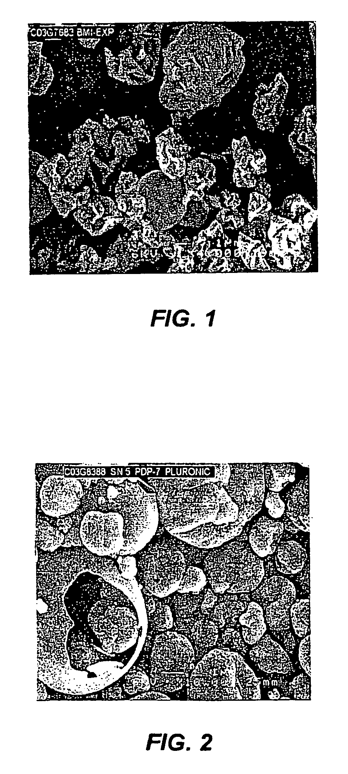

Solid particles of omega-interferon were obtained by spray drying IFN-ω with sucrose and methionine from 25 mM citrate solution with a solution concentration containing 3.3, 6.6, 3.3 and 7.1 mg/mL of IFN-ω, sucrose, methionine and citrate, respectively to give a final composition of 1:2:1:2.15 (IFN-ω: sucrose: methionine: citrate). The SEM image of the particles is shown in FIG. 1. The average particle size is 6.51 µm.

### EXAMPLE 2

Solid particles of IFN-ω with 1% Pluronic F68 surfactant was obtained by spray drying IFN-ω with sucrose and methionine and Pluronic F68 (Polyethylene oxide-PolyPropylene oxide copolymer) from 25 mM citrate solution with a solution concentration containing 3.3, 6.6, 3.3 7.1 and 0.2 mg/mL of IFN-ω, sucrose, methionine, citrate, and Pluronic F68, respectively to give a final composition of 1:2:1:2.15:0.06 (IFN-ω: sucrose: methionine: citrate: Pluronic F68). Addition of 1% of Pluronic F68 to the IFN-ω/excipient solution was successfully spray dried with a yield of approximately 49% at a batch size of 55 mL (1.1g solid). The SEM image of the particles is shown in FIG. 2. The particles have a smooth spherical shape. The average particle size is 4.03 µm.

### EXAMPLE 3

Four suspensions (A, B, C, and D) were prepared in the dry box and are listed in Table 4. The appropriate amount of SAIB was weighed and added into a scintillation glass vial. The appropriate amount of surfactant was added into the same vial if specified. The vial was heated to 50°C and hand mixed by a spatula. The appropriate amount of IFN- ω particles (as prepared in Example 1 or 2) was weighed and added into the vial. The vial was heated to 40°C or lower temperature. The suspension was mixed using a spatula.

**TABLE 4**

| | WEIGHT, % | WEIGHT, mg | TOTAL, g |
|---|---|---|---|
| A: Suspension (no surfactant) | | | |
| IFN-ω particles | 10 | 150 | |
| SAIB | 90 | 1350 | 1.5 |
| B: Suspension with 5% Pluronic F68 | | | |
| IFN-ω particles | 10 | 100 | |
| Pluronic F-68 | 5 | 50 | |
| SAIB | 85 | 850 | 1 |
| C: Suspension with 5% Span-40 | | | |
| IFN-ω particles | 10 | 100 | |
| Sorbitan monopalmitate (Span-40) | 5 | 50 | |
| SAIB | 85 | 850 | 1 |
| D: Particles with 1% Pluronic F68 in | | | |
| SAIB | 10 | 100 | |
| IFN-ω particles + 1% Pluronic F68 | 90 | 900 | 1 |
| SAIB | | | |

Stability samples of IFN-ω/SAIB suspension in PBS were obtained by weighing approximately 8 mg of EFN-ω/SAIB suspension into a 5 mL Vacutainer^{®} glass tube and adding 2 mL of PBS to the tube. The samples were stored at 37°C for stability testing. At each stability time point, the sample was taken out from the stability chamber. The liquid was decanted into a HPLC (High Performance Liquid Chromatrogram) vial and was analyzed directly by fast RP-HPLC (Reverse Phase High Performance Liquid Chromatography) method. For the SAIB gel, 0.5 mL of 50% ACN with 0.1% SDS was added into the tube, and the gel was dissolved for 60 minutes, then 2 mL of PBS was added to the tube. The cloudy solution was centrifuged and the liquid layer was transferred into the HPLC vial for fast RP-HPLC analysis. The protein recovery from liquid phase and solid phase and total recovery were calculated.

Stability samples of IFN-ω/SAIB suspension in implantable device reservoirs in PBS were set up as in Table 5. The samples were analyzed at initial, 3 days and 7 days.

**TABLE 5**

| Formulations (see Table 4) | Stability Sample Prep | | Sample Prep for Fast RP-HPLC Assay | | |
|---|---|---|---|---|---|
| | Suspension (mg) | PBS (mL) | Liquid | Solid (Gel) | |
| | | | | 50% ACN + 0.1% SDS | PBS (mL) |
| A | 8 | 0 | | 0.5 | 4 |
| A | 8 | 2 | Direct Analysis | 0.5 | 2 |
| B | 8 | 2 | Direct Analysis | 0.5 | 2 |
| C | 8 | 2 | Direct Analysis | 0.5 | 2 |
| D | 8 | 2 | Direct Analysis | 0.5 | 2 |

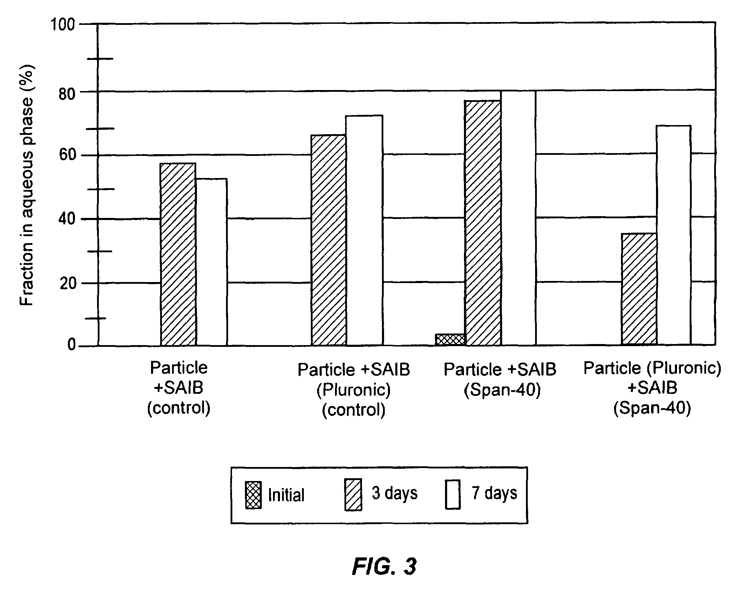

During the release of protein from suspension not all of the protein was instantaneously released into the release rate medium because SAIB is not water soluble. Recovery of the protein from the aqueous phase during select stability time points at 37°C is shown in FIG. 3. For formulation A (IFN-ω)/SAIB), fraction of protein recovered after 7 days is about 53%. For formulation B (IFN-ω/SAIB+Pluronic F68), fraction of protein recovered after 7 days is about 73%. For formulation C (IFN-ω/SAIB+Span-40), fraction of protein recovered after 7 days is about 80%. For formulation D (IFN-ω+Pluronic F68/SAIB), fraction of protein recovered after 7 days is about 69%. The results show that addition of surfactants into the SAIB vehicle or dry particle IFN-ω formulation enhanced release of IFN-ω into the aqueous phase after 7 days.

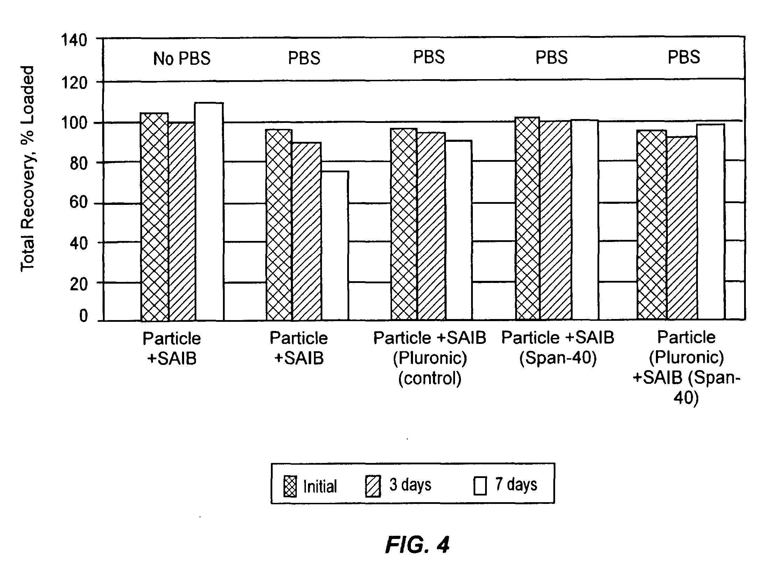

The total IFN-ω recovered from both aqueous phase and SAIB solid (gel) phase is shown in FIG. 4. For formulation A (without surfactant), the total recovery decreased over time, e.g., average drops of approximately 10% after 3 days and 25% after 7 days in PBS at 37°C were observed. Addition of surfactants into the suspension vehicle or dry particle formulation (formulations B-D) resulted in approximately 90-100% total recovery after about 7 days. The study shows that surfactants can be added into a dry particle IFN-ω formulation or suspension vehicle to enhance release of IFN-ω from the suspension vehicle into release rate medium.

## Claims

1. A suspension formulation that is deliverable via an implantable delivery device comprising:
a non-aqueous, hydrophobic vehicle exhibiting viscous fluid characteristics; and
a dry particle formulation comprising a biomolecule dispersed in the vehicle;
**characterized in that** a first surfactant is incorporated in the vehicle and a second surfactant is incorporated in the dry particle formulation.

2. The suspension formulation of claim 1, wherein the hydrophobic vehicle is non-polymeric.

3. The suspension formulation of claim 2, wherein the hydrophobic vehicle comprises substantially sucrose acetate isobutyrate.

4. The suspension formulation of claim 1, wherein the biomolecule is an interferon.

5. The suspension formulation of claim 4, which delivers the interferon from an implantable drug delivery device at 1 ng/day to 600 µg/day over at least one month.

6. The suspension formulation of claim 1, wherein the biomolecule is an interferon omega.

7. The suspension formulation of claim 1, wherein the biomolecule is spray dried with the surfactant.

8. The suspension formulation of claim 1, wherein the dry particle formulation further comprises one or more stabilizers and a buffer.

9. The suspension formulation of claim 8; wherein the stabilizers are selected from the group consisting of carbohydrate, antioxidant, and amino acid.

10. The suspension formulation of claim 1, wherein the hydrophobic vehicle is present in an amount greater than 60 wt%.

11. The suspension formulation of claim 1, wherein the dry particle formulation is present in a range from 0.01 to 40 wt%.

12. The suspension formulation of claim 1, wherein a surfactant loading in the dry particle formulation is in a range from 0 to 10 wt%.

13. The suspension formulation of claim 1, wherein a surfactant loading in the vehicle is in a range from 0 to 20 wt%.

14. A dry particle formulation comprising:
an interferon, a buffer, a surfactant, and one or more stabilizers selected from the group consisting of a carbohydrate, an antioxidant, and an amino acid.

15. The dry particle formulation of claim 14, which is spray dried.

16. The dry particle formulation of claim 14, wherein the interferon is interferon omega.

17. The dry particle formulation of claim 14, wherein the interferon is present in an amount ranging from 0.1 to 99.9 wt%.

18. The dry particle formulation of claim 17, wherein a weight ratio of each stabilizer to the interferon is in a range from 0.1 to 99.9.

19. The dry particle formulation of claim 18, wherein a weight ratio of each stabilizer to the interferon is greater than 0.5.

20. The dry particle formulation of claim 19, wherein a weight ratio of the carbohydrate to the interferon is greater than 1.0.

21. The dry particle formulation of claim 14, wherein a concentration of the buffer is in a range from 5 mM to 50 mM.

22. The dry particle formulation of claim 14, wherein a pH of the buffer is in a range from 5.0 to 8.0.

23. The dry particle formulation of claim 14, wherein interferon, carbohydrate, antioxidant and/or amino acid, buffer, and surfactant are present in a ratio of 1:2:1:1.5-2.5:0.06.

24. The dry particle formulation of claim 14, wherein the interferon is interferon omega, the carbohydrate is sucrose, the antioxidant and/or amino acid is methionine, and the buffer is citrate.

25. The dry particle formulation of claim 14, wherein the surfactant is present in a range from 0.01 to 10 wt%.

26. The dry particle formulation of claim 14, wherein the surfactant is present in a range from 0.01 to 5 wt%.

27. An implantable delivery device comprising:
a suspension formulation comprising a non-aqueous, hydrophobic vehicle exhibiting viscous fluid characteristics and a dry particle formulation comprising an interferon dispersed in the vehicle, **characterized in that** a first surfactant is incorporated in the vehicle and a second surfactant is incorporated in the dry particle formulation; and
a reservoir containing the suspension formulation in an amount sufficient to provide continuous delivery of the interferon in a therapeutically effective dose in an environment of use over at least one month.

28. A method of enhancing release of interferon omega in a hydrophilic release rate medium, comprising:
suspending a dry particle formulation of interferon omega in a non-aqueous, non-polymeric, hydrophobic vehicle; and
incorporating a first surfactant in the dry particle formulation and a second surfactant in the hydrophobic vehicle.

## Patentansprüche

1. Suspensionsformulierung, die über eine implantierbare Zufuhrvorrichtung zugeführt werden kann und Folgendes umfasst:
einen nicht-wässrigen, hydrophoben Träger, der viskose Fließeigenschaften aufweist, und
eine Trockenteilchenformulierung, umfassend ein in dem Träger dispergiertes Biomolekül,
**dadurch gekennzeichnet, dass** ein erstes Tensid in den Träger integriert ist und ein zweites Tensid in die Trockenteilchenformulierung integriert ist.

2. Suspensionsformulierung nach Anspruch 1, worin der hydrophobe Träger nicht polymer ist.

3. Suspensionsformulierung nach Anspruch 2, worin der hydrophobe Träger im Wesentlichen Saccharoseacetatisobutyrat umfasst.

4. Suspensionsformulierung nach Anspruch 1, worin das Biomolekül ein Interferon ist.

5. Suspensionsformulierung nach Anspruch 4, die das Interferon von einer implantierbaren Arzneimittelzufuhrvorrichtung aus in einer Rate von 1 ng/Tag bis 600 ng/Tag über zumindest einen Monat hinweg zuführt.

6. Suspensionsformulierung nach Anspruch 1, worin das Biomolekül ein Interferon-Omega ist.

7. Suspensionsformulierung nach Anspruch 1, worin das Biomolekül mit dem Tensid sprühgetrocknet ist.

8. Suspensionsformulierung nach Anspruch 1, worin die Trockenteilchenformulierung ferner einen oder mehrere Stabilisatoren und einen Puffer umfasst.

9. Suspensionsformulierung nach Anspruch 8, worin die Stabilisatoren aus der aus Kohlenhydraten, Antioxidationsmitteln und Aminosäuren bestehenden Gruppe ausgewählt sind.

10. Suspensionsformulierung nach Anspruch 1, worin der hydrophobe Träger in einer Menge von mehr als 60 Gew.-% vorhanden ist.

11. Suspensionsformulierung nach Anspruch 1, worin die Trockenteilchenformulierung in einer Menge im Bereich von 0,01 bis 40 Gew.-% vorhanden ist.

12. Suspensionsformulierung nach Anspruch 1, worin die Tensidbeladung in der Trockenteilchenformulierung im Bereich von 0 bis 10 Gew.-% liegt.

13. Suspensionsformulierung nach Anspruch 1, worin die Tensidbeladung in dem Träger im Bereich von 0 bis 20 Gew.-% liegt.

14. Trockenteilchenformulierung, umfassend:
ein Interferon, einen Puffer, ein Tensid und einen oder mehrere Stabilisatoren, die aus der aus Kohlenhydraten, Antioxidationsmitteln und Aminosäuren bestehenden Gruppe ausgewählt sind.

15. Trockenteilchenformulierung nach Anspruch 14, die sprühgetrocknet ist.

16. Trockenteilchenformulierung nach Anspruch 14, worin das Interferon Interferon-Omega ist.

17. Trockenteilchenformulierung nach Anspruch 14, worin das Interferon in einer Menge im Bereich von 0,1 bis 99,9 Gew.-% vorhanden ist.

18. Trockenteilchenformulierung nach Anspruch 17, worin das Gewichtsverhältnis jedes Stabilisators zu dem Interferon im Bereich von 0,1 bis 99,9 liegt.

19. Trockenteilchenformulierung nach Anspruch 18, worin das Gewichtsverhältnis jedes Stabilisators zu dem Interferon mehr als 0,5 beträgt.

20. Trockenteilchenformulierung nach Anspruch 19, worin das Gewichtsverhältnis des Kohlenhydrats zu dem Interferon mehr als 1,0 beträgt.

21. Trockenteilchenformulierung nach Anspruch 14, worin die Konzentration des Puffers im Bereich von 5 mM bis 50 mM liegt.

22. Trockenteilchenformulierung nach Anspruch 14, worin der pH des Puffers im Bereich von 5,0 bis 8,0 liegt.

23. Trockenteilchenformulierung nach Anspruch 14, worin das Interferon, das Kohlenhydrat, das Antioxidationsmittel und/oder die Aminosäure, der Puffer und das Tensid in einem Verhältnis von 1:2:1:1,5-2,5:0,06 vorhanden sind.

24. Trockenteilchenformulierung nach Anspruch 14, worin das Interferon Interferon-Omega ist, das Kohlenhydrat Saccharose ist, das Antioxidationsmittel und/oder die Aminosäure Methionin ist und der Puffer Citrat ist.

25. Trockenteilchenformulierung nach Anspruch 14, worin das Tensid in einer Menge im Bereich von 0,01 bis 10 Gew.-% vorhanden ist.

26. Trockenteilchenformulierung nach Anspruch 14, worin das Tensid in einer Menge im Bereich von 0,01 bis 5 Gew.-% vorhanden ist.

27. Implantierbare Zufuhrvorrichtung, umfassend:
eine Suspensionsformulierung, umfassend einen nicht-wässrigen, hydrophoben Träger, der viskose Fließeigenschaften aufweist, und eine Trockenteilchenformulierung, die ein in dem Träger dispergiertes Interferon umfasst, **dadurch gekennzeichnet, dass** ein erstes Tensid in den Träger integriert ist und ein zweites Tensid in die Trockenteilchenformulierung integriert ist, und
einen Behälter, der die Suspensionsformulierung in einer Menge enthält, die ausreicht, um eine kontinuierliche Zufuhr des Interferons in einer therapeutisch wirksamen Dosis in einem Anwendungsbereich über zumindest einen Monat hinweg bereitzustellen.

28. Verfahren zur Verbesserung der Freisetzung von Interferon-Omega in einem hydrophilen Freisetzungsmedium, umfassend:
das Suspendieren einer Trockenteilchenformulierung von Interferon-Omega in einem nicht-wässrigen, nicht polymeren, hydrophoben Träger und
das Integrieren eines ersten Tensids in die Trockenteilchenformulierung und eines zweiten Tensids in den hydrophoben Träger.

## Revendications

1. Formulation en suspension qui peut être administrée par le biais d'un dispositif d'administration implantable comprenant:
un excipient hydrophobe non aqueux présentant des caractéristiques fluidiques visqueuses ; et
une formulation particulaire sèche comprenant une biomolécule dispersée dans l'excipient ;
**caractérisée en ce qu'**un premier agent tensioactif est incorporé dans l'excipient et un second agent tensioactif est incorporé dans la formulation particulaire sèche.

2. Formulation en suspension selon la revendication 1, dans laquelle le véhicule hydrophobe est non polymère.

3. Formulation en suspension selon la revendication 2, dans laquelle l'excipient hydrophobe comprend sensiblement de l'acétate isobutyrate de saccharose.

4. Formulation en suspension selon la revendication 1, dans laquelle la biomolécule est l'interféron.

5. Formulation en suspension selon la revendication 4, qui administre l'interféron à partir d'un dispositif d'administration de médicament implantable à 1 ng/jour à 600 µg/jour sur au moins un mois.

6. Formulation en suspension selon la revendication 1, dans laquelle la biomolécule est un interféron oméga.

7. Formulation en suspension selon la revendication 1, dans laquelle la biomolécule est desséchée par nébulisation avec l'agent tensioactif.

8. Formulation en suspension selon la revendication 1, dans laquelle la formulation particulaire sèche comprend en outre un ou plusieurs stabilisants et un tampon.

9. Formulation en suspension selon la revendication 8, dans laquelle les stabilisants sont sélectionnés dans le groupe constitué par un glucide, un antioxydant et un acide aminé.

10. Formulation en suspension selon la revendication 1, dans laquelle l'excipient hydrophobe est présent en une quantité supérieure à 60 % en poids.

11. Formulation en suspension selon la revendication 1, dans laquelle la formulation particulaire sèche est présente dans une plage allant de 0,01 à 40 % en poids.

12. Formulation en suspension selon la revendication 1, dans laquelle une charge en agent tensioactif dans la formulation particulaire sèche se situe dans une plage allant de 0 à 10 % en poids.

13. Formulation en suspension selon la revendication 1, dans laquelle une charge en agent tensioactif dans l'excipient se situe dans une plage allant de 0 à 20 % en poids.

14. Formulation particulaire sèche comprenant :
l'interféron, un tampon, un agent tensioactif et un ou plusieurs stabilisants sélectionnés dans le groupe constitué par un glucide, un antioxydant et un acide aminé.

15. Formulation particulaire sèche selon la revendication 14, qui est desséchée par nébulisation.

16. Formulation particulaire sèche selon la revendication 14, dans laquelle l'interféron est l'interféron oméga.

17. Formulation particulaire sèche selon la revendication 14, dans laquelle l'interféron est présent en une quantité allant de 0,1 à 99,9 % en poids.

18. Formulation particulaire sèche selon la revendication 17, dans laquelle un rapport en poids de chaque stabilisant sur l'interféron se situe dans une plage allant de 0,1 à 99,9.

19. Formulation particulaire sèche selon la revendication 18, dans laquelle un rapport en poids de chaque stabilisant sur l'interféron est supérieur à 0,5.

20. Formulation particulaire sèche selon la revendication 19, dans laquelle un rapport en poids du glucide sur l'interféron est supérieur à 1,0.

21. Formulation particulaire sèche selon la revendication 14, dans laquelle une concentration du tampon se situe dans une plage de 5 mM à 50 mM.

22. Formulation particulaire sèche selon la revendication 14, dans laquelle un pH du tampon se situe dans une plage de 5,0 à 8,0.

23. Formulation particulaire sèche selon la revendication 14, dans laquelle l'interféron, le glucide, l'antioxydant et/ou l'acide aminé, le tampon et l'agent tensioactif sont présents dans un rapport de 1 : 2 : 1 : 1,5 à 2,5 : 0,06.

24. Formulation particulaire sèche selon la revendication 14, dans laquelle l'interféron est l'interféron oméga, le glucide est le saccharose, l'antioxydant et/ou l'acide aminé est la méthionine et le tampon est un citrate.

25. Formulation particulaire sèche selon la revendication 14, dans laquelle l'agent tensioactif est présent dans une plage allant de 0,01 à 10 % en poids.

26. Formulation particulaire sèche selon la revendication 14, dans laquelle l'agent tensioactif est présent dans une plage allant de 0,01 à 5 % en poids.

27. Dispositif d'administration implantable comprenant :
une formulation en suspension comprenant un excipient hydrophobe non aqueux présentant des caractéristiques fluidiques visqueuses et une formulation particulaire sèche comprenant l'interféron dispersé dans l'excipient, **caractérisé en ce qu'**un premier agent tensioactif est incorporé dans l'excipient et un second agent tensioactif est incorporé dans la formulation particulaire sèche; et
un réservoir contenant la formulation en suspension en une quantité suffisante pour assurer une administration continue de l'interféron en une dose thérapeutiquement efficace dans un environnement d'utilisation sur au moins un mois.

28. Procédé d'amélioration de la libération de l'interféron oméga dans un milieu de vitesse de libération hydrophile, comprenant :
la mise en suspension d'une formulation particulaire sèche d'interféron oméga dans un excipient hydrophobe non aqueux et non polymère ; et
l'incorporation d'un premier agent tensioactif dans la formulation particulaire sèche et d'un second agent tensioactif dans l'excipient hydrophobe.
